# EUROPEAN PATENT SPECIFICATION

(11) **EP 1 057 829 B1**
(45) Date of publication and mention of the grant of the patent: **20.11.2002**
(21) Application number: 99850097.9
(22) Date of filing: 04.06.1999
(51) Int. Cl.: C07D 487/04, A61K 31/519, C07D 498/04

(54) **Novel compounds and pharmaceutical compositions containing the same**
Kondensierte Pyrimidinderivate und diese enthaltende pharmazeutische Zubereitungen
Dérivés de la pyrimidine condensés et compositions pharmaceutiques les contenant

(43) Date of publication of application: 06.12.2000
(73) Proprietor: The Jordanian Pharmaceutical Manufacturing and Medical Equipment Co.Ltd., Jo-Naor 11710 (JO)
(72) Inventor: Badwan, Adnan Ali H., Amman 11185 (JO); El-Abadelah, Mustafa M.M., Amman (JO)
(74) Representative: Bjerre, Nils B.J.

(56) References cited:
- WO-A-98/49166

## Description

### Field of the Invention

The present invention relates to novel compounds, pharmaceutical compositions containing the same as well as use of said compounds in the manufacture of a medicament for treatment of erectile dysfunction.

### Background of the Invention

Erectile dysfunction is a disorder which is very common throughout the world. The recent introduction of sildenafil (the active ingredient in Viagra®) has improved the possibilities of treating this disorder significantly. Sildenafil and compounds closely related thereto are disclosed in EP 463 756, EP 702 555 and WO 98/49166 (all to Pfizer Ltd.).

However, despite the useful therapeutic properties of sildenafil, not all patients are successfully treated with this agent. Thus, there is still a great need in the art for compounds having improved therapeutic properties compared to sildenafil.

### Disclosure of the Invention

There are now provided novel compounds with surprisingly improved therapeutic efficiency in comparison with the prior art cited above. In summary, the present invention relates to a compound having the general formula (I): 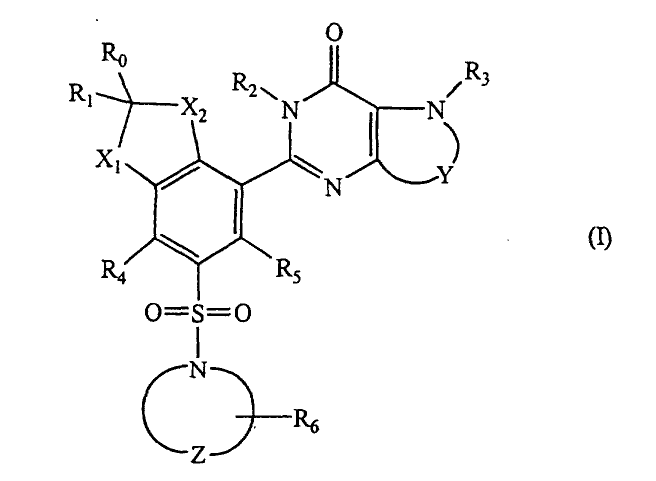 wherein R₀-R₆ are independently selected from at least one of a group of substituents (a)-(g) consisting of:
(a) H;
(b) straight chain, branched or cyclic saturated or unsaturated alkyl or hydroxyalkyl having 1-6 carbon atoms;
(c) O-alkyl, S-alkyl or N-(alkyl)ₙ, where alkyl is as defined in (b) and n is 1 or 2;
(d) C(O)-alkyl, O-C(O)-alkyl, S-C(O)-alkyl or NH-C(O)-alkyl, where alkyl is as defined in (b);
(e) F, Cl or Br;
(f) O-aryl;
(g) NR₈R₉, wherein R₈ and R₉ independently is H or straight chain, branched or cyclic saturated or unsaturated alkyl, C(O)-alkyl, hydroxyalkyl or O-alkyl having 1-6 carbons atoms; wherein NR₈R₉ optionally may form a five- or six-membered saturated or unsaturated ring;
wherein X₁ and X₂ are independently selected from a group of radicals consisting of:
-Cₘ- independently substituted with the substituents (a)-(g), where m is an integer from 1 to 3 and the radical -Cₘ- optionally may contain a double bond, ketone or thioketone functionality;
-O-;
-S-; and
-NR₁₀-, where R₁₀ is H or straight chain, branched or cyclic saturated or unsaturated alkyl,
C(O)-alkyl, hydroxyalkyl or O-alkyl having 1-6 carbons atoms;
wherein Y is selected from a group of radicals consisting of:
-CR₁₁=N-; -N=CR₁₂-; -N=N-; -CR₁₃=CR₁₄-; -CR₁₅R₁₆CR₁₇R₁₈-;
-CR₁₉R₂₀O-; -OCR₂₁R₂₂-; -CR₂₂R₂₃NR₂₄-; -NR₂₅CR₂₆R₂₇- and
-NR₂₈NR₂₉-, where R₁₁-R₂₉ are independently selected from the substituents (a)-(g);
wherein Z taken together with the nitrogen atom to which it is attached forms a group selected from pyrrolidinyl, piperidinyl, morpholinyl, imidazolyl, pyridinyl, pyrrolyl and 4-*N*-(R₃₀)-piperazinyl, whereby R₃₀ is selected from the substituents (a)-(g);
tautomers, solvates and radiolabelled derivatives thereof; and
pharmaceutically acceptable salts thereof.

As examples of pharmaceutically acceptable salts mention can be made of acid addition salts, *e.g.* a salt formed by reaction with hydrohalogen acids, such as hydrochloric acid, sulphuric acid, phosphoric acid, nitric acid, aliphatic, alicyclic, aromatic or heterocyclic sulphonic or carboxylic acids, such as formic acid, acetic acid, propionic acid, succinic acid, glycolic acid, lactic acid, malic acid, tartaric acid, citric acid, ascorbic acid, maleic acid, hydroxymaleic acid, pyruvic acid, *p*-hydroxybenzoic acid, embonic acid, methanesulphonic acid, ethanesulphonic acid, hydroxyethanesulphonic acid, halogenbenzensulphonic acid, toluenesulphonic acid and naphtalenesulphonic acid.

In a preferred embodiment of the present invention, Y is -CR₁₁=N-. R₁₁ is preferably an n-propyl group.

Furthermore, it is preferred that Z taken together with the nitrogen-atom to which it is attached forms a 4-*N*-(R₃₀)-piperazinyl group. Preferably, R₃₀ is a methyl group.

Moreover, it is preferred that X₁ is -Cₘ-. Preferably, m is 1. Most preferably, X₁ is -CH₂-.

It is preferred that X₂ is -O-.

In a more preferred embodiment of the present invention, R₂ is H.

In an even more preferred embodiment, R₃ is a methyl group.

In a still even more preferred embodiment, R₄, R₅ and R₆ are all H.

In the most preferred embodiment of the present invention, said compound is 5-[2,3-dihydro-5-(4-methylpiperazin-1-ylsulfonyl)-7-benzofuryl]-1-methyl-3-propyl-6,7-dihydro-1*H*-pyrazolo[4,3-d]pyrimidin-7-one, the structure of which is depicted hereinbelow. This compound is hereinafter denoted **7a**.

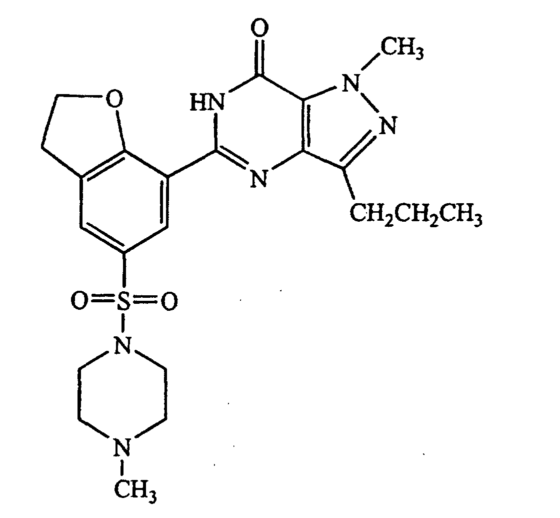

Furthermore, the present invention relates to a compound as set forth above for use as a pharmaceutical.

Accordingly, the present invention also relates to a pharmaceutical composition comprising a compound as set forth above as active ingredient in association with a pharmaceutically acceptable adjuvant, diluent or carrier.

The pharmaceutical composition may be adapted for oral, intravenous, topical, intraperitoneal, nasal, buccal, sublingual or subcutaneous administration or for administration via the respiratory tract in the form of *e.g.* an aerosol or an air-suspended fine powder. Thus, the composition may be in the form of *e.g.* tablets, capsules, powders, micro-particles, granules, syrups, suspensions, solutions, transdermal patches or suppositories.

It should be noted that the composition according to the present invention may optionally include two or more of the above outlined compounds.

In addition, the present invention relates to the use of a compound as outlined above for the manufacture of a medicament for treatment of erectile dysfunction.

Furthermore, it is also anticipated that the compounds according to the present invention have beneficial platelet anti-aggregatory, anti-vasospastic and vasodilatory activity. Thus, they should be useful in the treatment of a number of disorders, such as angina, hypertension, congestive heart failure, peripheral vascular disease, atherosclerosis, stroke, bronchitis, asthma, allergic rhinitis and glaucoma.

The typical dosage of the compounds according to the present invention varies within a wide range and will depend on various factors such as the individual requirement of each patient and the route of administration. The dosage is generally within the range of 0.01-100 mg/kg body weight.

The general synthetic pathway to formula (I) may be summarized as shown below (Δ=heat) :

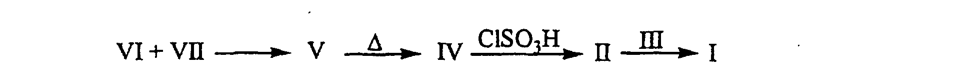

Thus, the present invention also relates to a process for the preparation of a compound as set forth above, wherein a compound having the general formula (II) is reacted with a compound having the general formula (III), optionally in the presence of a solvent, wherein R₀-R₆ and X-Z are as defined above.

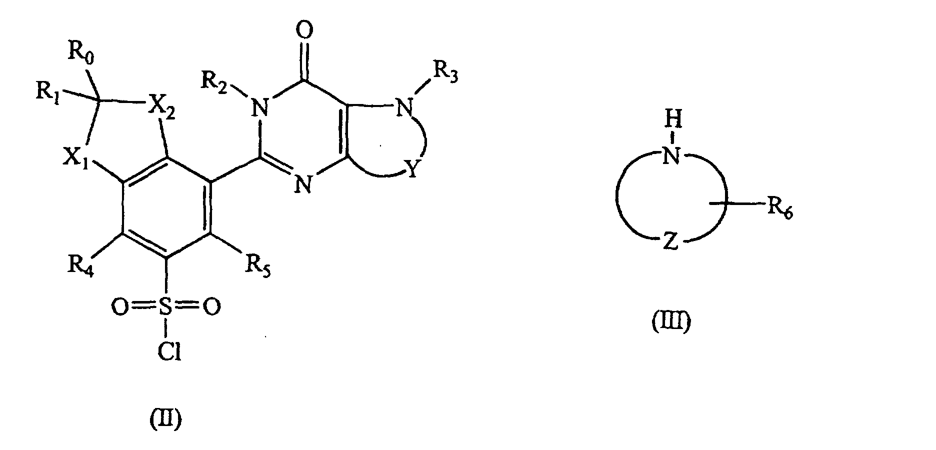

The compound (II) is prepared by reacting a compound having the general formula (IV) with ClSO₃H, optionally in the presence of a solvent.

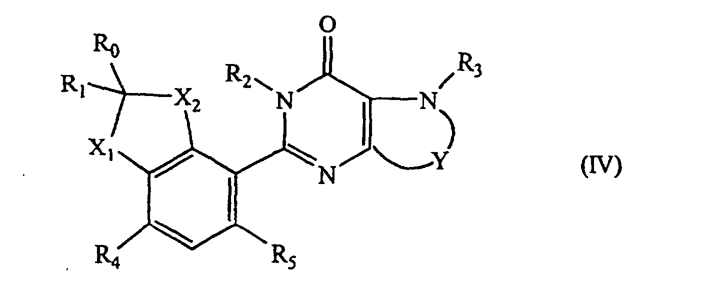

The compound (IV) is prepared by heating a compound having the general formula (V) under basic conditions, optionally in the presence of a solvent.

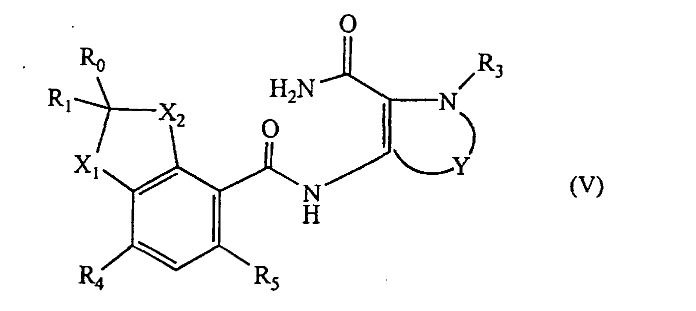

The compound (V) is prepared by reacting a compound having the general formula (VI) with a compound having the general formula (VII), optionally in the presence of a solvent and a base.

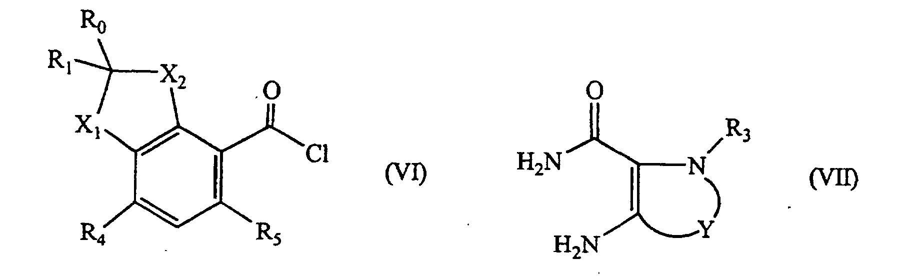

As for the selection of *e.g.* suitable reaction and purification conditions, useful guidance is also provided by the following publications :
DeWald, H.A., Nordin, I.C., L'Italien, Y.J., Parcell, R.F., *J. Med. Chem.*, **16**, 1346-1354 (1973);
Meyers, A.I., Reuman, M., Gabel., R.A., *J. Org. Chem.*, **46,** 783-788 (1981);
Högberg, T., de Paulis, T., Johansson, L., Kumar, Y., Hall, H., Ögren, S.O., *J. Med. Chem.*, **33,** 2305-2309 (1990).

By guidance of known reference literature, the synthesis of the starting substances (VI) and (VII) is readily accomplished by a person skilled in the art.

The present invention is further illustrated by the following non-limiting experimental part.

### Brief Description of the Drawings

Fig. 1 shows a comparative study of total erection episodes as a function of dose for rats treated with **7a** and sildenafil, respectively.

Fig. 2 shows a comparative study of penile erection index as a function of dose for rats treated with **7a** and sildenafil, respectively.

### Preparation of the compounds of the present invention

Instruments used for analysis:

The melting points (m.p.) were determined on an electrothermal Mel-Temp. apparatus. They are uncorrected. ¹H and ¹³C NMR spectra were recorded on a Bruker-WM 400 or -DPX 300 MHz spectrometer, with tetramethylsilane (TMS) as internal reference. Electron impact (EI) mass spectra were obtained using a Finnigan 731 spectrometer at 70 eV. Elemental analyses were performed at the Microanalytical Laboratory of the Chemistry Department, Al-Najah National University, West Bank.

### Example 1: Preparation of compound 4.

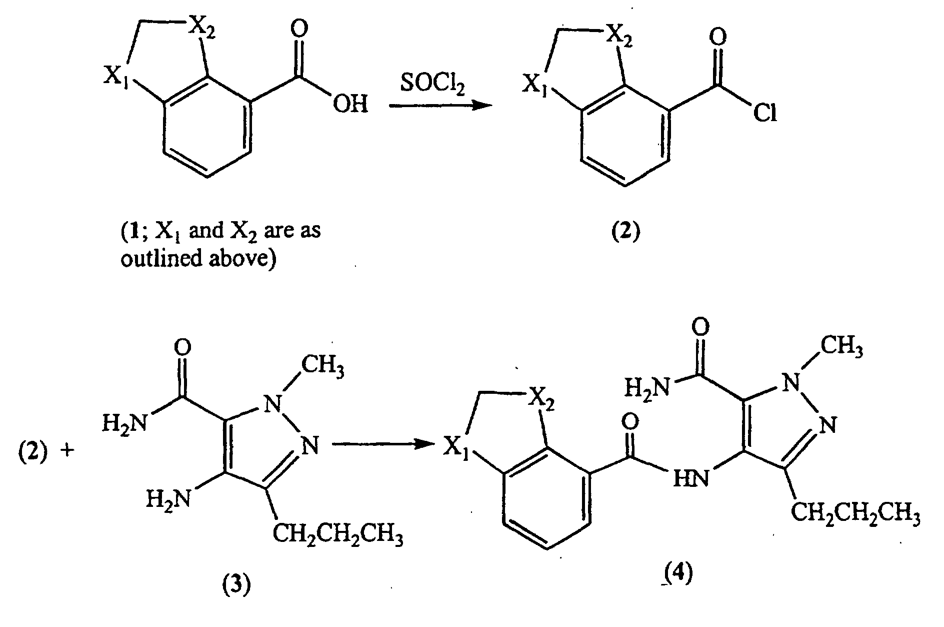

Compound **4** was prepared by treating **1** (0.1 mole) with SOCl₂ in a conventional manner yielding **2**, which was then refluxed with **3** in benzene (100 ml) and NEt₃ (30 ml) for 2-3 h. The benzene was distilled off, and the solid product **4** was collected, washed with H₂O, dried and recrystallized from a suitable solvent. Yield: 82-93%.

### Example 2: Preparation of compound 5.

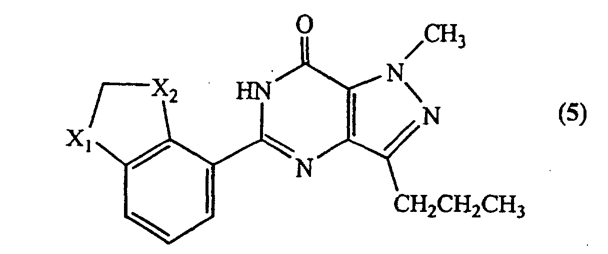

Potassium t-butoxide (0.01 mole) was added to a stirred suspension of **4** (0.01 mole) in t-BuOH (60 ml), and the resulting mixture was refluxed for 8 h. Water (40 ml) was then added, after which the solution was neutralized with diluted HCl (aq; 4%) to pH 7 and cooled. The solid product **5** was collected, washed with cold H₂O and recrystallized from a suitable solvent. The yield was 86-95%.

### Example 3: Preparation of compound 6.

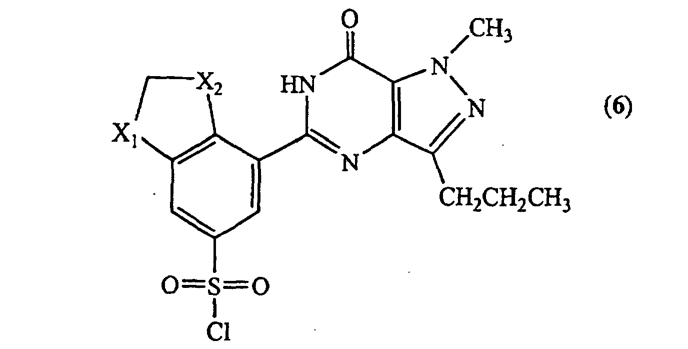

Compound **5** (0.006 mole) was added in portions to chlorosulfonic acid (4 ml) cooled to 0°C under stirring. The temperature of the reaction mixture was then allowed to rise to 25°C, followed by heating to 65-70°C for 1 h. The reaction mixture was subsequently poured onto crushed ice (50 g), after which the precipitated solid product **6** was collected and used directly in the next reaction step. Yield: 82-91%.

### Example 4: Preparation of compound 7.

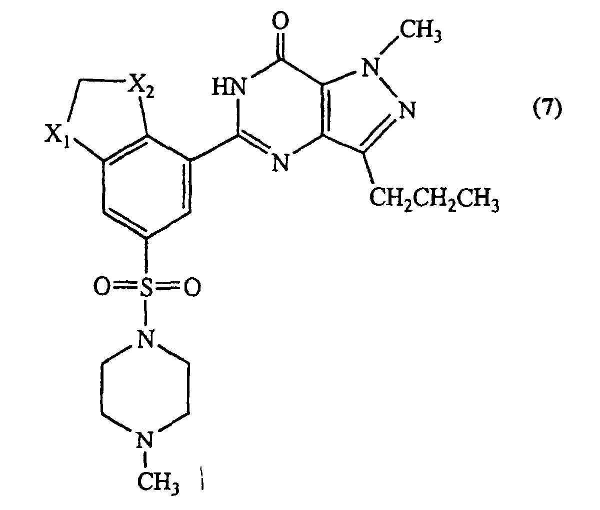

Compound **6** (0.005 mole) dissolved in THF (20 ml) was added to a solution of 1-methylpiperazine (2 ml) in THF (20 ml). The resulting mixture was stirred for 1 h at 20-25°C. The THF was distilled off, and the residue was treated with cold H₂O. The resulting white solid product **7** was collected, washed with H₂O, drained and recrystallized from a suitable solvent. Yield: 80-88%.

By following the reaction protocol above, the compounds **7a**-**7I** listed in Table 1 below were prepared.

**Table 1:**

| Compounds prepared, where X₁ is as specified and X₂ is -O- for all the compounds. | |
|---|---|
| Compound | X₁ |
| **7a** | CH₂ |
| **7b** | O |
| **7c** | S |
| **7d** | NCH₃ |
| **7e** | NC₂H₅ |
| **7f** | NCH(CH₃)₂ |
| **7g** | NC(O)CH₃ |
| **7h** | NC(O)NHPh |
| **7i** | NC(S)NHPh |
| **7j**^{*i)*} | C=O |
| **7k**^{*i)*} | C=S |
| **7l**^{*ii)*} | NH |

| | |
|---|---|
| *i)* The compounds **7j** and **7k** were obtained after protection/deprotection of a 3-keto/thioketo group in the corresponding compounds **2j** and **2k.** | |
| *ii)* Compound **7l** was obtained from **7g** by selective hydrolysis in 15% HCl for 20 min with heating. | |

Example 5: Detailed preparation and physical properties of **7a** and its precursors.

### Preparation of 4-(2,3-dihydro-7-benzofurylamino)-1-methyl-3-propyl-5-pyrazole-carboxamide (4a, i.e. 4 wherein X₁=CH₂ and X₂=O) :

A mixture of 2,3-dihydrobenzofuran-7-carboxylic acid (1.5 g, 0.0091 mole) and SOCl₂ (8 ml) was refluxed (oil bath) for 3 h. Excess of SOCl₂ was removed *in vacuo,* and the residual acid chloride was treated with a solution of compound **1** (1.4 g, 0.0077 mole) in anhydrous benzene (25 ml), followed by addition of NEt₃ (3 ml). The solid residue was soaked in cold water (40 ml), and the remaining solid product was collected by suction filtration, drained, washed with water (2 x 20 ml) and diethyl ether (2 x 10 ml) and dried, thereby yielding **4a**. Product yield = 2.3 g (91%);
M.p. = 173-174°C;
Elemental analysis = Calculated for C₁₇H₂₀N₄O₃ (MW=328.37) C 62.18, H 6.14, N 17.06%. Found C 61.95, H 6.07, N 17.11%.
¹H NMR (CDCl₃) : δ 0.86 (t, J=7.4 Hz, 3H, CH₂CH₂C*H*₃), 1.56 (m, 2H, CH₂C*H*₂CH₃), 2.46 (t, J=7.6 Hz, 2H, C*H*₂CH₂CH₃), 3.27 (t, J=8.5 Hz, 2H, C3'-H), 3.97 (s, 3H, N-C*H*₃), 4.73 (t, J=8.5 Hz, 2H, C2'-H), 6.94 (t, J=7.6 Hz, 1H, C5'-H), 7.34 (d, J=7.2 Hz, 1H, C4'-H), 6.26, 7.72 (2 br s, 1H each of CON*H*₂), 7.86 (d, J=8.1 Hz, 1H, C6'-H), 8.88 (br s, 1H, N*H*CO).
¹³C NMR (CDCl₃): δ 13.7 (CH₂CH₂*C*H₃), 22.2 (CH₂*C*H₂CH₃), 27.5 (*C*H₂CH₂CH₃), 28.9 (C-3'), 39.1 (N-*C*H₃), 72.8 (C-2'), 114.5 (C-3), 115.6 (C-7'), 121.4 (C-5'), 128.0 (C-3'a), 129.35, 129.34 (C-4' and C-6'), 132.1 (C-4), 147.0 (C-5), 157.9 (C-7'a), 161.7 (NH*C*O), 165.8 (*C*ONH₂).

### Preparation of 5-(2,3-dihydro-7-benzofuryl)-1-methyl-3-propyl-6,7-dihydro-1H-pyrazolo[4,3-d]pyrimidine-7-one (5a):

Potassium t-butoxide (0.5 g, 0.0045 mole) was added to a stirred suspension of compound **4a** (1.1 g, 0.0034 mole) in t-butanol (20 ml), and the resulting mixture was heated under reflux (oil bath) for 8 h and then allowed to cool to room temperature. Water (14 ml) was added, after which the solution was neutralized with HCl (aq; 4%; 13 ml) to pH 7, cooled to about 5-10°C, collected by suction filtration, washed with cold water (2 x 10 ml), crystallized from ethanol and dried, thereby yielding **5a**. Product yield = 1.0 g (96%);
M.p. = 176-178°C (decomposition);
Elemental analysis = Calculated for C₁₇H₁₈N₄O₂ (MW=310.36) C 65.79, H 5.85, N 18.05%. Found C 65.72, H 5.91, N 17.93%.
¹H NMR (CDCl₃): δ 0.99 (t, J=7.4 Hz, 3H, CH₂CH₂C*H*₃), 1.82 (m, 2H, CH₂C*H*₂CH₃), 2.86 (t, J=7.6 Hz, 2H, C*H*₂CH₂CH₃), 3.22 (t, J=8.1 Hz, 2H, C3'-H), 4.19 (s, 3H, N-C*H*₃), 4.73 (t, J=8.1 Hz, 2H, C2'-H), 6.94 (t, J=7.6 Hz, 1H, C5'-H), 7.22 (d, J=7.2 Hz, 1H, C4'-H), 8.16 (d, J=8.1 Hz, 1H, C6'-H), 10.69 (br s, 1H, N6-H).
¹³C NMR (CDCl₃) : δ 14.0 (CH₂CH₂*C*H₃), 22.2 (CH₂*C*H₂CH₃), 27.7 (*C*H₂CH₂CH₃), 28.9 (C-3'), 38.1 (N-*C*H₃), 72.6 (C-2'), 114.5 (C-3), 121.6 (C-5'), 124.4 (C-7'), 127.3, 127.5 (C-4' and C-6'), 128.1 (C-3'a), 138.5 (C-3a), 146.4 (C-5), 146.7 (C-7a), 154.0 (C-7), 156.8 (C-7'a).

### Preparation of 5-(2,3-dihydro-5-chlorosulfonyl-Z-benzofuryl)-1-methyl-3-propyl-6,7-dihydro-1H-pyrazolo[4,3-d]pyrimidin-7-one (6a) :

Compound **5a** (0.95 g, 0.003 mole) was added in portions to chlorosulfonic acid (2 ml) cooled to 0°C (ice-bath) under stirring. The resulting yellow solution was then allowed to attain room temperature and was subsequently slowly heated to 65-70°C (oil bath) for 1 h. The reaction mixture was then slowly poured onto crushed ice (25 g), whereby a white solid precipitated immediately. The white solid was filtered, dried and recrystallized from THF/petroleum ether (b.p. 40-60°C), thereby yielding **6a**.
Product yield = 1.04 g (84%);
M.p. = 221-222°C.
No elemental analysis was performed on **6a** (C₁₇H₁₇ClN₄O₄S; MW=408.86).
[M]⁺=408/410 (3:1 ratio; Cl isotopic peaks);
The crude product **6a** (92% yield; m.p. 216-218°C) can be used directly in the next reaction step.

### Preparation of 5-[2,3-dihydro-5-(4-methylpiperazin-1-ylsulfonyl)-7-benzofuryl]-1-methyl-3-propyl-6,7-dihydro-1H-pyrazolo[4,3-d]pyrimidin-7-one (7a):

Compound **6a** (1.25 g, 0.003 mole) was dissolved in THF (10 ml) and added to a solution of 1-methylpiperazine (1 ml) in THF (10 ml). The resulting mixture was stirred at room temperature for 1 h. The THF was then removed *in vacuo*, and the residue was treated with cold water (50 ml). The resulting white precipitate was filtered under suction, washed with water (2 x 10 ml), drained and recrystallized from 90% ethanol, thereby yielding **7a**. Product yield = 1.2 g (83%);
M.p. = 194-195°C;
Elemental analysis = Calculated for C₂₂H₂₈N₆O₄S (MW=472.57) C 55.92, H 5.97, N 17.78, S 6.79%. Found C 56.00, H 6.09, N 17.51, S 6.73%.
¹H NMR (CDCl₃): δ 0.96 (t, J=7.2 Hz, 3H, CH₂CH₂C*H*₃), 1.79 (m, 2H, CH₂C*H*₂CH₃), 2.20 (s, 3H, N4"-C*H*₃), 2.43 (br s, 4H, C3"-H/C5"-H), 2.85 (t, J=7.2 Hz, 2H, C*H*₂CH₂CH₃), 3.01 (br s, 4H, C2"-H/C6"-H), 3.32 (t, J=8.5 Hz, 2H, C3'-H), 4.17 (s, 3H, N1-C*H*₃), 4.89 (t, J=8.5 Hz, 2H, C2'-H), 7.56 (s, 1H, C4'-H), 8.54 (s, 1H, C6'-H), 10.49 (br s, 1H, N6-H).
¹³C NMR (CDCl₃): δ 13.9 (CH₂CH₂*C*H₃), 22.1 (CH₂*C*H₂CH₃), 27.5 (*C*H₂CH₂CH₃), 28.4 (C-3'), 38.1 (N-*C*H₃), 45.6 (N4"-*C*H₃), 45.9 (C-3"/C-5"), 53.9 (C-2"/C-6"), 74.0 (C-2'), 114.5 (C-3), 124.4 (C-7'), 126.2 (C-4'), 128.5 (C-6'), 129.1 (C-5'), 130.2 (C-3'a), 138.1 (C-3a), 145.2 (C-5), 146.7 (C-7a), 153.6 (C-7), 159.9 (C-7'a).

### Animal experiments involving compound 7a

The purpose of this study was to compare the biological activity of compound **7a** with that of sildenafil. In particular, the respective ED₅₀-value (ED=effective dose), erection episodes and penile erection indices of said compounds in the treatment of male rats were determined. The penile erection index is an established means of determining the erection promoting properties of a substance (see *e.g.* Ang, H.H., Sim, M.K., *Pharm. Sci.*, **3**, 117-119 (1997) and references cited therein).

In these experiments, the compounds **7a** and sildenafil were administered to male rats orally. The doses used for both drugs were 0.0781, 0.1562, 0.3125 and 0.625 mg/kg body weight. Sildenafil was dissolved in distilled water, whereas **7a** was dissolved in 1% HCl solution (aq). Control animals were administered with the vehicles only, *i.e.* distilled water or the 1% HCl solution. During the experiments, the rats were placed in glass cages for observation and had access to food and water. During 2 h after administration of the investigated compounds, the penile erection of the rats was monitored. It is worth mentioning that no copulation mounting behaviour was observed in these experiments.

The number of rats responding to this experiment protocol was recorded, and the ED₅₀ results are shown in Table 2.

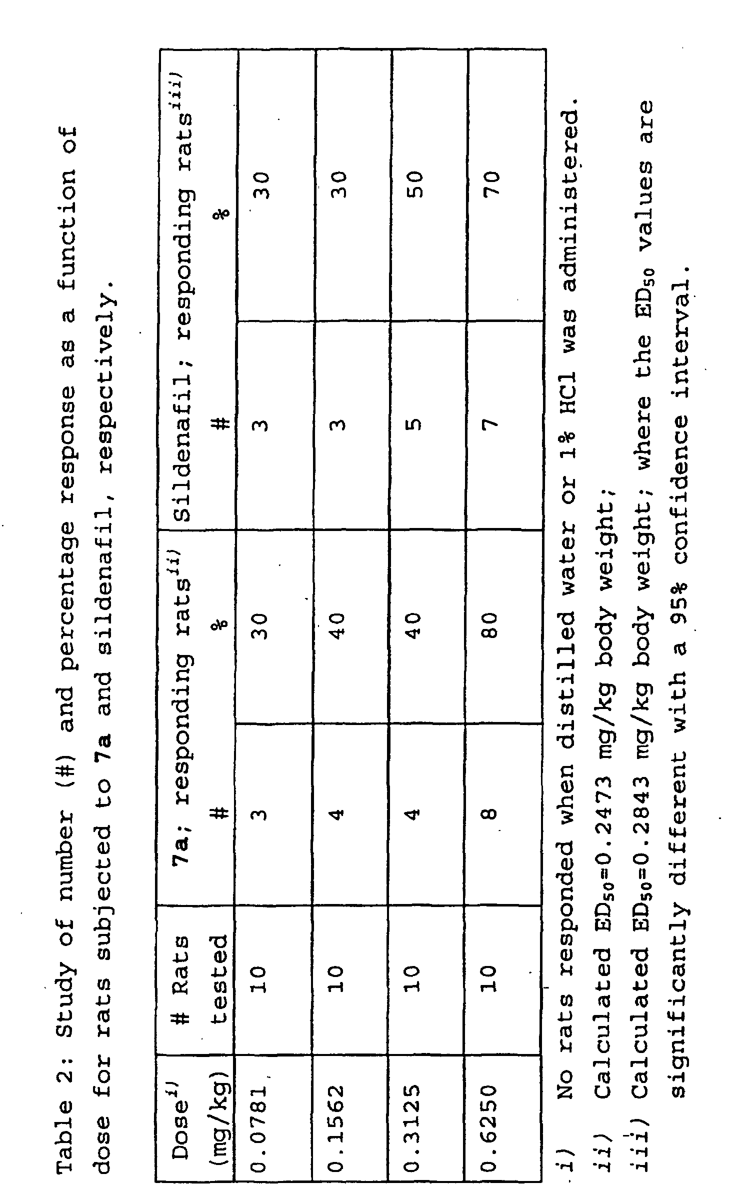

As is clear from Table 2, the ED₅₀ value of **7a** is lower than that of sildenafil. Thus, a lower dose of **7a** as compared to sildenafil is required in order to elicit an erectile response.

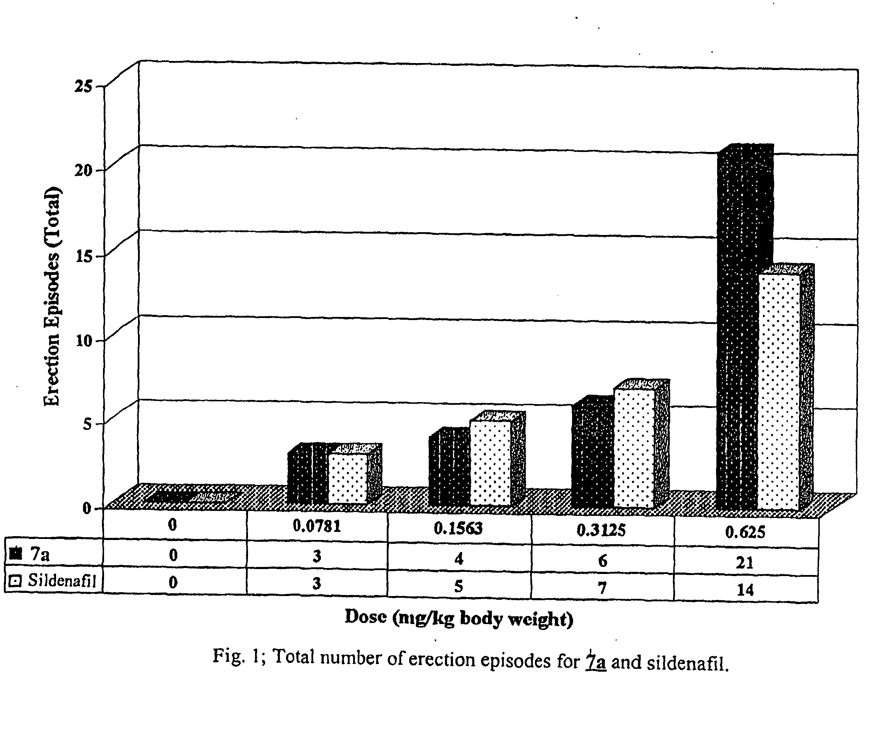

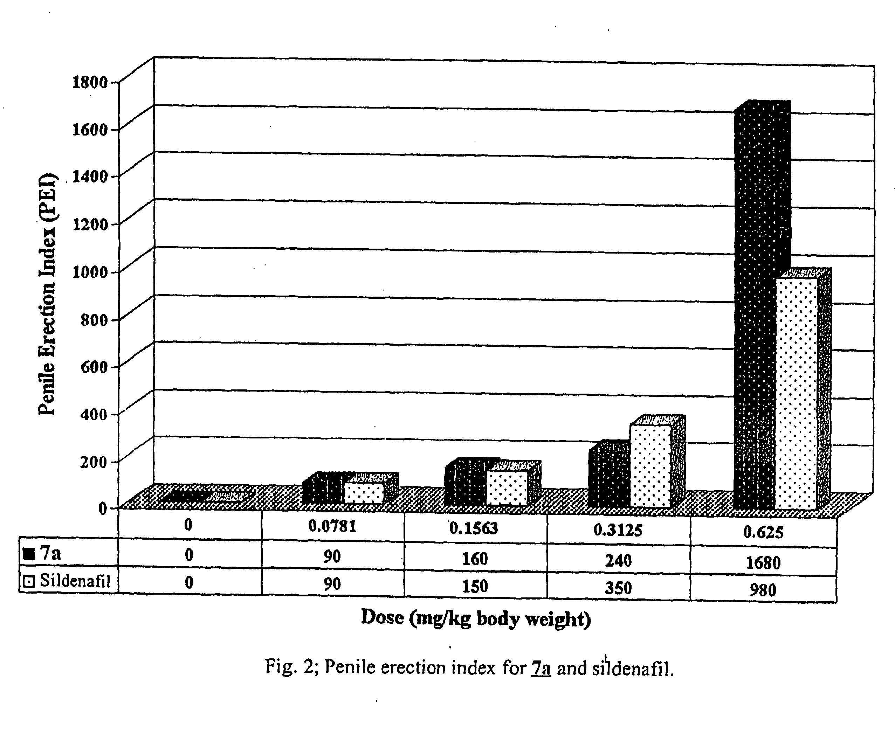

Furthermore, as for the intensity of the erectile response *per se,* the observed number of erection episodes and calculated penile erection indices substantiate that the compound **7a** is superior to sildenafil, especially at higher doses. The total number of observed erection episodes and the calculated penile erection indices are depicted in Figs 1 and 2, respectively.

Moreover, according to preliminary toxicity studies in rats, the compound **7a** is tolerated up to a dose of about 35 mg/100 kg body weight without any detrimental effects. The compound **7a** appears to be completely nontoxic and free from undesirable side-effects. Thus, high doses of **7a** provide a particularly efficient means for treatment of erectile dysfunction.

In summary, it should be clear from the present disclosure that the compounds according to the present invention are versatile new pharmaceutically active agents for the treatment of erectile dysfunction.

## Claims

1. A compound having the general formula (I): 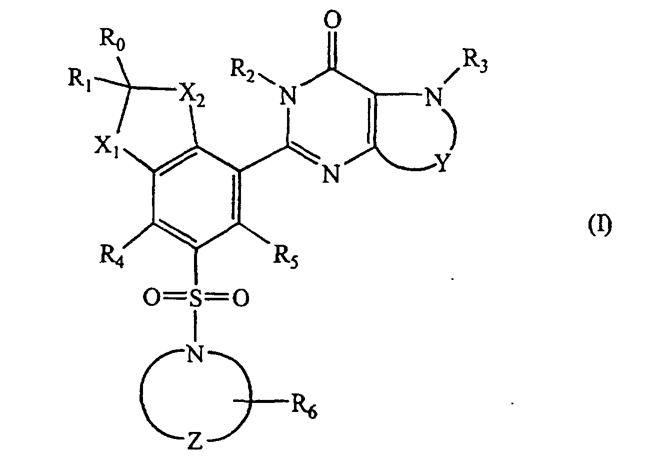 wherein R₀-R₆ are independently selected from at least one of a group of substituents (a)-(g) consisting of:
(a) H;
(b) straight chain, branched or cyclic saturated or unsaturated alkyl or hydroxyalkyl having 1-6 carbon atoms;
(c) O-alkyl, S-alkyl or N-(alkyl)ₙ, where alkyl is as defined in (b) and n is 1 or 2;
(d) C(O)-alkyl, O-C(O)-alkyl, S-C(O)-alkyl or NH-C(O)-alkyl, where alkyl is as defined in (b);
(e) F, Cl or Br;
(f) O-aryl;
(g) NR₈R₉, wherein R₈ and R₉ independently is H or straight chain, branched or cyclic saturated or unsaturated alkyl, C(O)-alkyl, hydroxyalkyl or O-alkyl having 1-6 carbons atoms;
wherein NR₈R₉ optionally may form a five- or six-membered saturated or unsaturated ring;
wherein X₁ and X₂ are independently selected from a group of radicals consisting of:
-Cₘ- independently substituted with the substituents (a)-(g), where m is an integer from 1 to 3 and the radical -Cₘ- optionally may contain a double bond, ketone or thioketone functionality;
-O-;
-S-; and
-NR₁₀-, where R₁₀ is H or straight chain, branched or cyclic saturated or unsaturated alkyl,
C(O)-alkyl, hydroxyalkyl or O-alkyl having 1-6 carbons atoms;
wherein Y is selected from a group of radicals consisting of:
-CR₁₁=N-; -N=CR₁₂-; -N=N-; -CR₁₃=CR₁₄-; -CR₁₅R₁₆CR₁₇R₁₈-;
-CR₁₉R₂₀O-; -OCR₂₁R₂₂-; -CR₂₂R₂₃NR₂₄-; -NR₂₅CR₂₆R₂₇- and
-NR₂₈NR₂₉-, where R₁₁-R₂₉ are independently selected from the substituents (a)-(g);
wherein Z taken together with the nitrogen atom to which it is attached forms a group selected from pyrrolidinyl, piperidinyl, morpholinyl, imidazolyl, pyridinyl, pyrrolyl and 4-*N*-(R₃₀)-piperazinyl, whereby R₃₀ is selected from the substituents (a)-(g);
tautomers, solvates and radiolabelled derivatives thereof; and
pharmaceutically acceptable salts thereof.

2. A compound according to claim 1, wherein Y is -CR₁₁=N-.

3. A compound according to claim 2, wherein R₁₁ is an n-propyl group.

4. A compound according to any one of claims 1-3, wherein Z taken together with the nitrogen atom to which it is attached forms a 4-*N*-(R₃₀)-piperazinyl group.

5. A compound according to claim 4, wherein R₃₀ is a methyl group.

6. A compound according to any one of claims 1-5, wherein X₁ is -Cₘ-.

7. A compound according to claim 6, wherein m is 1.

8. A compound according to claim 7, wherein X₁ is -CH₂-.

9. A compound according to any one of claims 1-8, wherein X₂ is -O-.

10. A compound according to any one of claims 1-9, wherein R₂ is H.

11. A compound according to claim 10, wherein R₃ is a methyl group.

12. A compound according to claim 11, wherein R₄, R₅ and R₆ are H.

13. A compound according to claim 12, wherein said compound is 5-[2,3-dihydro-5-(4-methylpiperazin-1-ylsulfonyl)-7-benzofuryl]-1-methyl-3-propyl-6,7-dihydro-1*H*-pyrazolo[4,3-d]pyrimidin-7-one.

14. A compound according to any one of claims 1-13 for use as a pharmaceutical.

15. A pharmaceutical composition comprising a compound according to any one of claims 1-13 as active ingredient in association with a pharmaceutically acceptable adjuvant, diluent or carrier.

16. Use of a compound according to any one of claims 1-13 for the manufacture of a medicament for treatment of erectile dysfunction.

17. A process for the preparation of a compound having the general formula I as defined in any one of claims 1-13, whereby a compound having the general formula (II) is reacted with a compound having the general formula (III), optionally in the presence of a solvent, 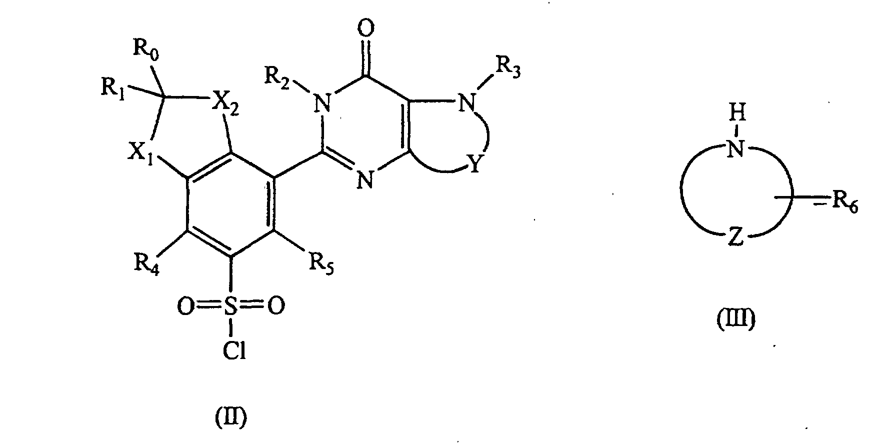 wherein R₀-R₆ and X-Z are as defined in any one of claims 1-13.

18. A process according to claim 17, whereby the compound (II) is prepared by reacting a compound having the general formula (IV) with ClSO₃H, optionally in the presence of a solvent.

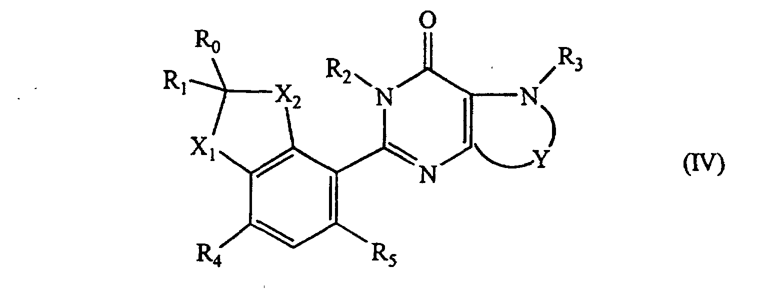

19. A process according to claim 18, whereby the compound (IV) is prepared by heating a compound having the general formula (V) under basic conditions, optionally in the presence of a solvent.

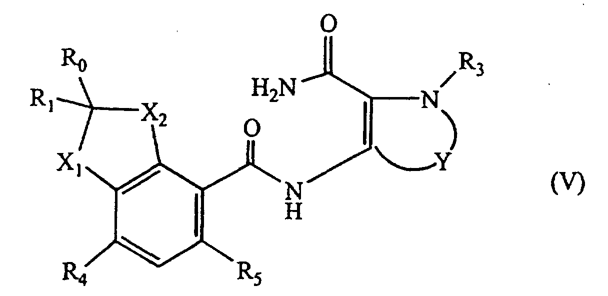

20. A process according to claim 19, whereby the compound (V) is prepared by reacting a compound having the general formula (VI) with a compound having the general formula (VII), optionally in the presence of a solvent and a base.

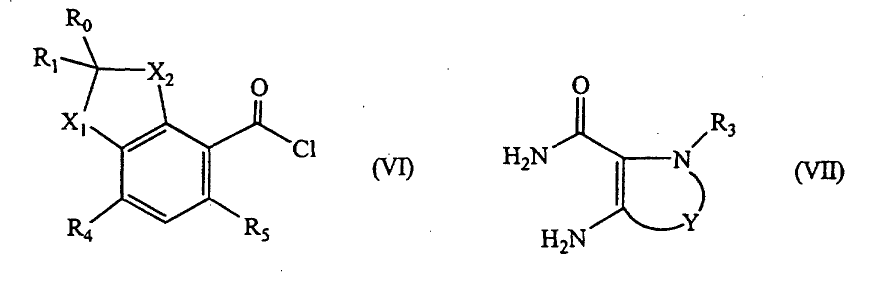

## Patentansprüche

1. Verbindung mit der allgemeinen Formel (l): 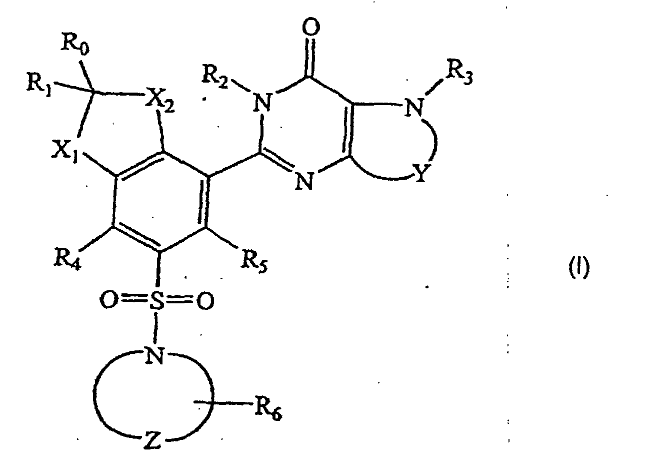 worin R₀-R₆ unabhängig ausgewählt werden aus mindestens einem von einer Gruppe von Substituenten (a) - (g) bestehend aus:
(a) H;
(b) geradkettigem, verzweigtem oder cyclischem gesättigtem oder ungesättigtem Alkyl oder Hydroxyalkyl mit 1-6 Kohlenstoffatomen;
(c) O-Alkyl, S-Alkyl oder N-(Alkyl)ₙ, wobei Alkyl wie in (b) definiert ist und n 1 oder 2 ist;
(d) C(O)-Alkyl, O-C(O)-Alkyl, S-C(O)-Alkyl oder NH-C(O)-Alkyl, wobei Alkyl wie in (b) definiert ist;
(e) F, Cl oder Br;
(f) O-Aryl;
(g) NR₈R₉, worin R₈ und R₉ unabhängig H oder geradkettiges, verzweigtes oder cyclisches gesättigtes oder ungesättigtes Alkyl, C(O)-Alkyl, Hydroxyalkyl oder O-Alkyl mit 1-6 Kohlenstoffatomen ist;
wobei NR₈R₉ gegebenenfalls einen fünf- oder sechsgliedrigen gesättigten oder ungesättigten Ring bilden kann;
worin X₁ und X₂ unabhängig ausgewählt werden aus einer Gruppe von Resten, bestehend aus:
-Cₘ- unabhängig substituiert mit den Substituenten (a) - (g), wobei m eine ganze Zahl von 1 bis 3 ist und der Rest -Cₘ- gegebenenfalls eine Doppelbindung, Keton- oder Thioketonfunktionalität enthalten kann;
-O-;
-S-; und
-NR₁₀-, wobei R₁₀ H oder geradkettiges, verzweigtes oder cyclisches gesättigtes oder ungesättigtes Alkyl, C(O)-Alkyl, Hydroxyalkyl oder O-Alkyl mit 1-6 Kohlenstoffatomen ist;
worin Y ausgewählt wird aus einer Gruppe von Resten, bestehend aus:
-CR₁₁=N-; -N=CR₁₂-; -N=N-; -CR₁₃=CR₁₄-; -CR₁₅R₁₆CR₁₇R₁₈-; -CR₁₉R₂₀O-;
-OCR₂₁R₂₂-; -CR₂₂R₂₃NR₂₄-; -NR₂₅CR₂₆R₂₇- und -NR₂₈NR₂₉-, wobei R₁₁-R₂₉ unabhängig ausgewählt werden aus den Substituenten (a) - (g);
worin Z zusammen mit dem Stickstoffatom, an das es gebunden ist, eine Gruppe, ausgewählt aus Pyrrolidinyl, Piperidinyl, Morpholinyl, Imidazolyl, Pyridinyl, Pyrrolyl und 4-*N*-(R₃₀)-Piperazinyl bildet, wobei R₃₀ ausgewählt wird aus den Substituenten (a) - (g);
Tautomere, Solvate und radioaktiv markierte Derivate davon; und
pharmazeutisch annehmbare Salze davon.

2. Verbindung nach Anspruch 1, worin Y -CR₁₁=N- ist.

3. Verbindung nach Anspruch 2, worin R₁₁ eine n-Propylgruppe ist.

4. Verbindung nach einem der Ansprüche 1-3, worin Z zusammen mit dem Stickstoffatom, an das es gebunden ist, eine 4-*N*-(R₃₀)-Piperazinylgruppe bildet.

5. Verbindung nach Anspruch 4, worin R₃₀ eine Methylgruppe ist.

6. Verbindung nach einem der Ansprüche 1-5, worin X₁ -Cₘ- ist.

7. Verbindung nach Anspruch 6, worin m 1 ist.

8. Verbindung nach Anspruch 7, worin X₁ -CH₂- ist.

9. Verbindung nach einem der Ansprüche 1-8, worin X₂ -O- ist.

10. Verbindung nach einem der Ansprüche 1-9, worin R₂ H ist.

11. Verbindung nach Anspruch 10, worin R₃ eine Methylgruppe ist.

12. Verbindung nach Anspruch 11, worin R₄, R₅ und R₆ H sind.

13. Verbindung nach Anspruch 12, worin die Verbindung 5-[2,3-Dihydro-5-(4-methylpiperazin-1-ylsulfonyl)-7-benzofuryl]-1-methyl-3-propyl-6,7-dihydro-1*H*-pyrazolo-[4,3-d]pyrimidin-7-on ist.

14. Verbindung nach einem der Ansprüche 1-13 zur Verwendung als Pharmazeutikum.

15. Pharmazeutische Zusammensetzung, umfassend eine Verbindung nach einem der Ansprüche 1-13 als Wirkstoff in Verbindung mit einem pharmazeutisch annehmbaren Adjuvans, Verdünnungsmittel oder Träger.

16. Verwendung einer Verbindung nach einem der Ansprüche 1-13 zum Herstellen eines Arzneimittels für die Behandlung einer erektilen Dysfunktion.

17. Verfahren zum Herstellen einer Verbindung mit der allgemeinen Formel (I), wie sie in einem der Ansprüche 1-13 definiert ist, wobei eine Verbindung mit der allgemeinen Formel (II) mit einer Verbindung mit der allgemeinen Formel (III), gegebenenfalls in Gegenwart eines Lösungsmittels, umgesetzt wird, 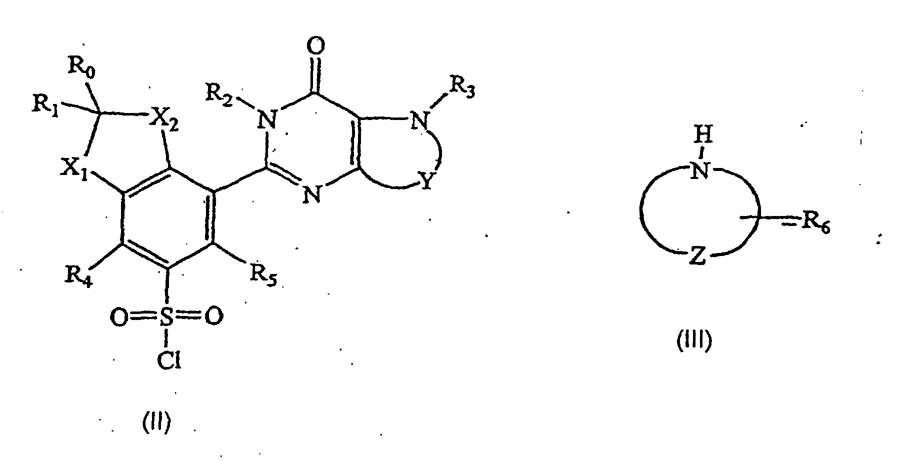 worin R₀-R₆ und X-Z wie in einem der Ansprüche 1-13 definiert sind.

18. Verfahren nach Anspruch 17, wobei die Verbindung (II) durch Umsetzen einer Verbindung mit der allgemeinen Formel (IV) mit CISO₃H, gegebenenfalls in Gegenwart eines Lösungsmittels, hergestellt wird.

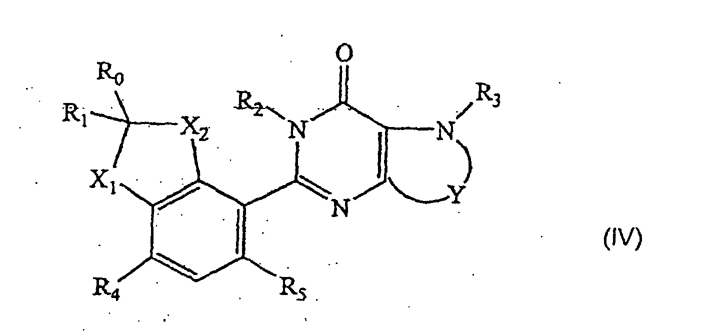

19. Verfahren nach Anspruch 18, wobei die Verbindung (IV) durch Erwärmen einer Verbindung der allgemeinen Formel (V) unter basischen Bedingungen, gegebenenfalls in Gegenwart eines Lösungsmittels, hergestellt wird.

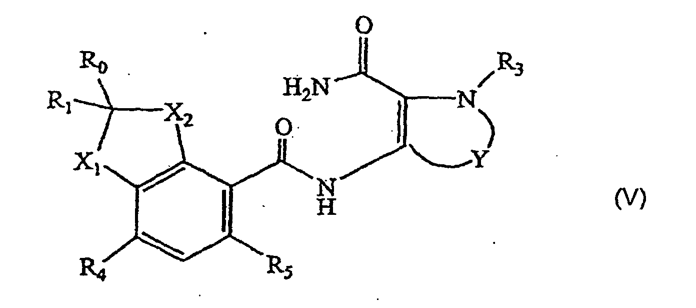

20. Verfahren nach Anspruch 19, wobei die Verbindung (V) durch Umsetzen einer Verbindung mit der allgemeinen Formel (VI) mit einer Verbindung mit der allgemeinen Formel (VII), gegebenenfalls in Gegenwart eines Lösungsmittels und einer Base, hergestellt wird.

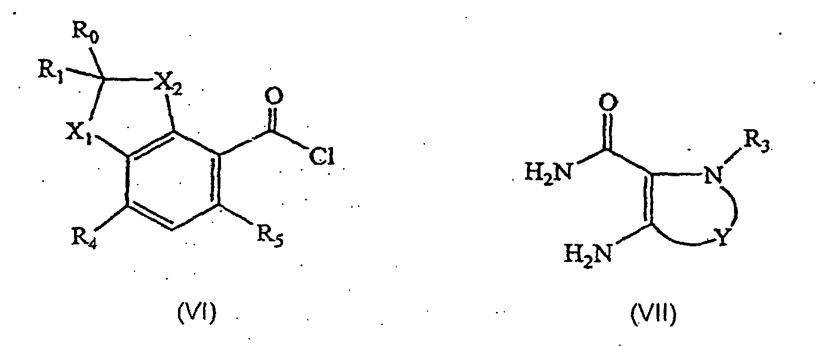

## Revendications

1. Composé ayant la formule générale (I) : 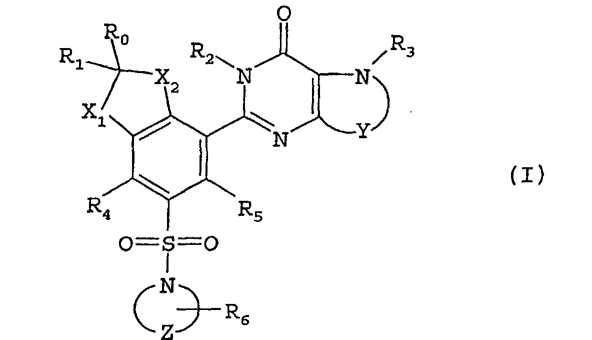 dans laquelle R₀ à R₆ sont choisis indépendamment dans l'un au moins des groupes de substituants (a) à (g) constitués de :
(a) H ;
(b) alkyle, ou hydroxyalkyle à chaîne droite ou ramifiée ou cyclique saturé ou insaturé ayant de 1 à 6 atomes de carbone ;
(c) O-alkyle, S-alkyle ou N-(alkyle)ₙ, où l'alkyle est tel que défini en (b) et n vaut 1 ou 2 ;
(d) C(O)-alkyle, O-C(O)-alkyle, S-C(O)-alkyle ou NH-C(O)-alkyle, où l'alkyle est tel que défini en (b) ;
(e) F, Cl ou Br ;
(f) O-aryle ;
(g) NR₈R₉, où R₈ et R₉ sont indépendamment H ou alkyle, C(O)-alkyle, hydroxyalkyle ou O-alkyle à chaîne droite ou ramifiée ou cyclique saturé ou insaturé, ayant de 1 à 6 atomes de carbone ; NR₈R₉ pouvant le cas échéant former un cycle saturé ou insaturé à cinq ou six termes ;
dans laquelle X₁ et X₂ sont choisis indépendamment dans un groupe de radicaux constitué de :
- Cₘ- substitué indépendamment par les substituants (a) à (g) , où m est un nombre entier valant de 1 à 3 et le radical -Cₘ- peut le cas échéant contenir une double liaison ou bien une fonctionnalité cétone ou thiocétone ;
-O- ;
-S- ; et
-NR₁₀-, où R₁₀ est H ou un alkyle, C(O)-alkyle, hydroxyalkyle ou O-alkyle à chaîne droite ou ramifiée ou cyclique saturé ou insaturé, ayant de 1 à 6 atomes de carbone ;
dans laquelle Y est choisi dans un groupe de radicaux constitué de :
-CR₁₁=N-, -N=CR₁₂-, -N=N-, -CR₁₃=CR₁₄-, -CR₁₅R₁₆CR₁₇R₁₈-, -CR₁₉R₂₀O-, -OCR₂₁R₂₂-, -CR₂₂R₂₃NR₂₄-, -NR₂₅CR₂₆R₂₇- et -NR₂₈NR₂₉-, où R₁₁ à R₂₉ sont choisis indépendamment parmi les substituants (a) à (g) ;
dans laquelle Z, pris ensemble avec l'atome d'azote auquel il est rattaché, forme un groupe choisi parmi les pyrrolidinyle, pipéridinyle, morpholinyle, imidazolyle, pyridinyle, pyrrolyle et 4-*N*-(R₃₀)-pipérazinyle, où R₃₀ est choisi parmi les substituants (a) à (g) ;
ainsi que des tautomères, des solvates et des dérivés radiomarqués de celui-ci ; et
des sels pharmaceutiquement acceptables de celui-ci.

2. Composé selon la revendication 1, dans lequel Y est -CR₁₁=N-.

3. Composé selon la revendication 2, dans lequel R₁₁ est un groupe n-propyle.

4. Composé selon l'une quelconque des revendications 1 à 3, dans lequel Z, pris ensemble avec l'atome d'azote auquel il est rattaché, forme un groupe 4-*N*-(R₃₀)-pipérazinyle.

5. Composé selon la revendication 4, dans lequel R₃₀ est un groupe méthyle.

6. Composé selon l'une quelconque des revendications 1 à 5, dans lequel X₁ est -Cₘ-.

7. Composé selon la revendication 6, dans lequel m vaut 1.

8. Composé selon la revendication 7, dans lequel X₁ est -CH₂-.

9. Composé selon l'une quelconque des revendications 1 à 8, dans lequel X₂ est -O-.

10. Composé selon l'une quelconque des revendications 1 à 9, dans lequel R₂ est H.

11. Composé selon la revendication 10, dans lequel R₃ est un groupe méthyle.

12. Composé selon la revendication 11, dans lequel R₄, R₅ et R₆ sont H.

13. Composé selon la revendication 12, dans lequel ledit composé est la 5-[2,3-dihydro-5-(4-méthylpipérazin-1-ylsulfonyl)-7-benzofuryl]-1-méthyl-3-propyl-6,7-dihydro-1H-pyrazolo[4,3-d]pyrimidin-7-one.

14. Composé selon l'une quelconque des revendications 1 à 13, destiné à être utilisé comme produit pharmaceutique.

15. Composition pharmaceutique comprenant un composé selon l'une quelconque des revendications 1 à 13 en tant que principe actif en association avec un adjuvant, un diluant ou un véhicule pharmaceutiquement acceptable.

16. Utilisation d'un composé selon l'une quelconque des revendications 1 à 13 pour fabriquer un médicament destiné au traitement des troubles de l'érection.

17. Procédé de préparation d'un composé ayant la formule générale I tel que défini dans l'une quelconque des revendications 1 à 13, dans lequel un composé ayant la formule générale (II) est mis à réagir avec un composé ayant la formule générale (III), le cas échéant en présence d'un solvant, 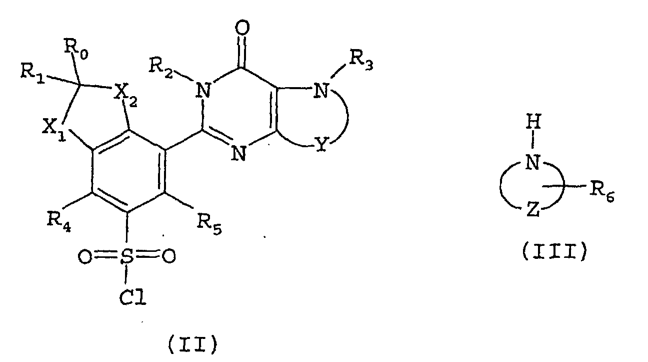 où R₀ à R₆ et X à Z sont tels que définis dans l'une quelconque des revendications 1 à 13.

18. Procédé selon la revendication 17, dans lequel le composé (II) est préparé en faisant réagir un composé ayant la formule générale (IV) avec du ClSO₃H, le cas échéant en présence d'un solvant.

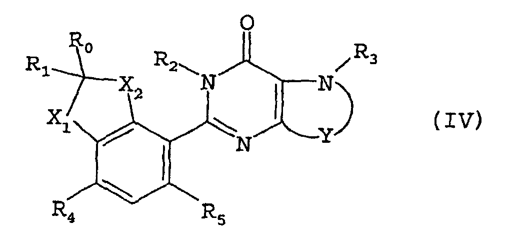

19. Procédé selon la revendication 18, dans lequel le composé (IV) est préparé en chauffant un composé ayant la formule générale (V) dans des conditions basiques, le cas échéant en présence d'un solvant.

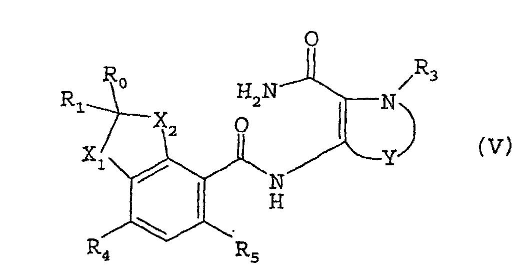

20. Procédé selon la revendication 19, dans lequel le composé (V) est préparé en faisant réagir un composé ayant la formule générale (VI) avec un composé ayant la formule générale (VII), le cas échéant en présence d'un solvant et d'une base.
